# EUROPÄISCHE PATENTANMELDUNG

(11) **EP 2 243 823 A2**
(43) Veröffentlichungstag der Anmeldung: **27.10.2010**
(21) Anmeldenummer: 09010728.5
(22) Anmeldetag: 20.08.2009
(51) Int. Cl.: C12M 1/107, C12P 5/02

(54) **Verfahren und Vorrichtung zur Erzeugung und Verteilung von Energie**

(30) Priorität: 20.08.2008 DE 102008038502
(71) Anmelder: Freinecker, Dieter, 84028 Landshut (DE)
(72) Erfinder: Freinecker, Dieter, 84028 Landshut (DE)
(74) Vertreter: Pausch, Thomas Ernst

(57) **Zusammenfassung**

Die Erfindung betrifft ein Verfahren und eine Vorrichtung zur Erzeugung und Verteilung von Energie, bei dem vorzugsweise lignocellulosehaltige Rohstoffe verwendet werden, wobei in einer ersten Station die Rohstoffe (1) angeliefert werden, und in dieser ersten Station eine Vorbehandlung und Aufbereitung (2) dieser Rohstoffe (1) zu einem flüssigen Gärsubstrat erfolgt, in einer zweiten, von der ersten räumlich getrennten, Station das Gärsubstrat einem Biovergasungsprozess (3) zugeführt wird zu einer Herstellung von Biogas (4), und in einer dritten Station Energie verschiedener Energieformen (4a, 4b, 4c) aus dem Biogas (4) gewonnen wird.

## Beschreibung

Die Erfindung bezieht sich auf ein Verfahren und eine Vorrichtung zur Erzeugung und Verteilung von Energie unter Verwendung lignocellulosehaltiger Rohstoffe nach dem Oberbegriff der Ansprüche 1 und 6.

Die Herstellung von Energie aus Energiepflanzen und von Energie aus Biogas ist angesichts der stetig steigenden Öl- und Gaspreise eine zunehmend interessante Art der Energiegewinnung. Auch im Hinblick auf Umweltschutzaspekte erscheint eine Energiegewinnung aus Energiepflanzen und/oder aus Biogas mit einer neutralen CO₂-Bilanz als zukunftsträchtige Methode. Verschiedene Verfahren bzw. Vorrichtungen zur Herstellung von Biogas oder von Energie aus Biogas sind in den letzten Jahren entwickelt worden.

Die DE 10 2006 012 130 A1 zeigt ein Verfahren für die Gewinnung von Biogas aus cellulosehaltigen Rohstoffen, bei dem aus einem Gärsubstrat einer die Mikroorganismen enthaltenden Reaktoreinheit ein Substrat und eine Flüssigkeit aufgegeben wird, wobei während einer im Reaktor ablaufenden Hydrolyse eine Aufspaltung des Gärsubstrats unter Bildung von Intermediärprodukten erfolgt, und die Intermediärprodukte im wesentlichen zu kurzkettigen Fettsäuren und die Fettsäuren zu Biogas abgebaut werden. Dieses Verfahren zur Herstellung von Biogas erlaubt die Verwendung cellulosehaltiger Rohstoffe unter Verzicht auf Gülle, wodurch die Biogasproduktion von der Tierhaltung entkoppelt werden kann. Jedoch weist dieses Verfahren keinen Vorbehandlungsschritt der Rohstoffe auf, weshalb einige Nachteile in Kauf genommen werden: Schlecht aufbereitete Faserstoffe lassen nur geringe Raumbelastungen zu, es kommt zu einer Schwimmdeckenbildung und/oder zu einer Verspinnung der Fasern und somit zu einer schlechten Durchmischung. Überdies ist durch mangelnde Vorbehandlung des Rohstoffes nur mit niedrigen Abbauraten zu rechnen, was sich in einer niedrigeren Energieausbeute bei gleichzeitig hohen Verweilzeiten niederschlägt. Um ausreichend hohe Gasmengen zu erzeugen, müssen große Volumina zur Vergärung bereitgestellt werden. Insgesamt ist der Energiegewinn bezogen auf den Energieeinsatz beim Einbringen von cellulosehaltigen Rohstoffen gering, der Nutzungsgrad des Verfahrens bzw. der Anlage ist niedrig.

Die Herstellung von Energie aus Biogas ist meist an landwirtschaftliche Betriebe bzw. Kommunen gekoppelt, da die notwendigen Gärsubstrate oft Abfälle aus solchen Betrieben bzw. Kommunen sind: Bioabfall, Gülle, Fette oder Pflanzen. Diese Stoffe werden, ehe es zur Biovergasung kommt, an den Biogasanlagen gelagert, was, oftmals gekoppelt mit einem fahrlässigen Umgang mit diesen Stoffen, in vielen Fällen zu einer massiven Geruchsbelästigung vor Ort führt. Dies mag ein Grund für geäußerte Vorbehalte der Bevölkerung gegenüber der Biogastechnologie sein. Durch die Inhomogenität der Rohstoffe ist es zudem schwierig, den Gärungsprozess ideal einzustellen, so dass die tatsächlich produzierte Gasmenge weit unter der theoretisch möglichen liegt. Aus den vorgenannten Gründen wird eine industrielle Energiegewinnung aus Biogas derzeit als noch nicht wirtschaftlich angesehen.

Der Erfindung liegt die Aufgabe zugrunde, ein industriell anwendbares Verfahren und eine Vorrichtung zur Erzeugung und Verteilung von Energie aus Biogas zur Verfügung zu stellen, bei welchen cellulosehaltige Rohstoffe verwertet werden, welches eine hohe Energieausbeute bei gleichzeitig guter öffentlichen Akzeptanz bietet.

Diese Aufgabe wird durch ein Verfahren nach Anspruch 1 und eine Vorrichtung nach Anspruch 6 gelöst.

Bei dem erfindungsgemäßen Verfahren zur Erzeugung und Verteilung von Energie unter Verwertung von lignocellulosehaltigen Rohstoffen ist vorgesehen, dass in einer ersten Station die Rohstoffe angeliefert werden, und bereits in dieser ersten Station eine Vorbehandlung und eine Aufbereitung dieser Rohstoffe zu einem flüssigen Gärsubstrat erfolgt, und in einer zweiten Station das Gärsubstrat einem Biovergasungsprozess zugeführt wird zu einer Herstellung von Biogas, und in einer dritten Station das aus dem Biovergasungsprozess gewonnene Biogas in eine über größere Strecken transportierbare, bzw. leitbare Energieform gewandelt wird, insbesondere elektrische Energie, Wärmeenergie, oder chemische Energie.

Durch die erfindungsgemäße Vorbehandlung und Aufbereitung in der ersten Station werden üblicherweise beim Biovergasungsprozess auftretende Probleme in der Hydraulik und der Prozesstechnik, wie beispielsweise eine Schwimmdeckenbildung, bereits in der ersten Station behoben: Eine zielgerichtete Vorbehandlung und Aufbereitung führt zu einer besserer stofflichen Homogenität des Gärsubstrates, und daraus ergeben sich kürzere Verweilzeiten sowie bessere Abbauraten und höhere Ausbeuten. Der so erzielbare kontinuierliche Prozess macht in weiterer Folge den Einsatz von effektiveren Biogasreaktoren möglich: Biogasanlagen können bei gleicher Leistung kleiner ausgeführt werden, der Flächenbedarf wird geringer, es werden weniger Substrate bei gleicher Leistung benötigt. So bleiben schließlich am Ende weniger zu entsorgende Reststoffe übrig, und es kommt zu einer Reduzierung des Eigenenergieverbrauchs. Durch die Verwendung eines flüssigen Gärsubstrates wird die Lagerungsproblematik an der Biogasanlage entschärft, die Geruchsemissionen werden stark vermindert, und damit wird die Akzeptanz für Biogasanlagen in der Bevölkerung erhöht.

Dem Prinzip der Erfindung folgend ist hierbei vorgesehen, dass die zweite Station von der ersten räumlich getrennt ist. Diese Trennung der Vorbehandlung und der Aufbereitung vom eigentlichen Biogasprozess eröffnet eine wirtschaftliche industrielle Biogaserzeugung. Der Transport des Gärsubstrates kann über Rohrleitungen erfolgen oder vermittels üblicher Transportmittel wie beispielsweise Lastkraftwagen.

Nach der Erfindung können die Rohstoffe aus cellulose- und lignocellulosehaltiger Biomasse jeglicher Form bestehen, insbesondere Gräser und/oder Körner und/oder Wasserschnitt und/oder Algen und/oder Schilf und/oder Holz und/oder Holzabfälle und/oder Rinde und/oder tierische Exkremente aufweisen. Damit können beispielsweise Stoffe verwertet werden, die ansonsten für keine Weiterverarbeitung geeignet sind.

In einer bevorzugten Ausgestaltung der Erfindung kann vorgesehen sein, dass die Vorbehandlung und die Aufbereitung in der ersten Station im wesentlichen ein Sortieren, ein Befreien von Verunreinigungen, ein Aufschließen der Rohstoffe und eine biologische Behandlung umfasst. Damit kann ein für die Biogaserzeugung ideales Stoffgemenge erreicht werden.

Das erfindungsgemäße Verfahren zeichnet sich in einer Weiterbildung dadurch aus, dass das Aufschließen der Rohstoffe ein Zerkleinern und ein Zerfasern ist, wobei die Oberfläche der derart gewonnenen Rohstoffpartikel um das 2 bis 1000-fache vergrößert wird gegenüber der Oberfläche des ursprünglichen Rohstoffes. Des Weiteren kann vorgesehen sein, dass die Rohstoffe auf eine Länge von 0,1 mm bis 10 mm zerkleinert werden, vorzugsweise auf 0,2 mm bis 3 mm. Vermittels dieser Maßnahmen kann eine ausreichend große Angriffsfläche geschaffen werden für die bei der Biovergasung (Hydrolyse und Biogenese) aktiv tätigen verschiedenen Bakterienstämme.

Bei einer vorteilhaften Ausbildung der Erfindung kann vorgesehen sein, dass die im Biomasserohstoff eingelagerten Proteine vor oder nach der Vorbehandlung rückgewonnen werden. Die Proteine können in der pharmazeutischen und/oder in der chemischen Industrie verwendet werden.

Erfindungsgemäß sieht die Proteinrückgewinnung vor, dass der geschnittene und gemahlene Rohstoff ausgepresst wird. Der derart gewonnene Saft wird gereinigt und auf maximal 90°C erhitzt, mit einem anschließenden Dekantieren der Proteine. Das Filtrat kann in verwertender Weise dem eigentlichen Biogasprozess zugeführt werden. Die Proteinrückgewinnung kann vor oder nach der Vorbehandlung erfolgen.

In einer bevorzugten Weiterbildung der Erfindung kann in einer die Rohstoffe schonender Weise vorgesehen sein, dass die Rohstofftemperatur während des Vorbehandlungsprozesses im Eintrittsbereich zwischen -10°C und +50°C liegt und zwischen +10°C und +90°C im Austrittsbereich, vorzugsweise zwischen +5°C und +50°C im Eintrittsbereich und zwischen +35°C und +85°C im Austrittsbereich.

Nach der Erfindung kann die Aufbereitung der vorbehandelten Rohstoffe durch hydrolytische Verflüssigung erfolgen. Diese hydrolytische Verflüssigung kann durch Enzyme und/oder durch thermophile Bakterienkulturen herbeigeführt werden. Eine Verflüssigung durch Enzyme ermöglicht eine gezielte Dosierung und einen preiswerten Einsatz aufgrund ihrer kommerziellen Verfügbarkeit. Thermophile Bakterienkulturen produzieren die Enzyme vor Ort, es ist keine permanente Zugabe zu den Rohstoffen erforderlich.

Erfindungsgemäß kann vorgesehen sein, dass der Feststoffgehalt bei der Aufbereitung im Bereich von 5% bis 50% bezogen auf die Trockensubstanzmasse des verwendeten Rohstoffes liegt. Damit läßt sich eine optimale Biogasausbeute bei der Vergärung erzielen.

In einer weiteren bevorzugten konstruktiven Ausgestaltung der Erfindung kann der pH-Wert des Gärsubstrates während der Biovergasung zwischen 4 und 12 liegen, vorzugsweise zwischen 6 und 8. Dies schafft ein optimales Milieu für die hydrolytische Verflüssigung.

Für das Erzielen einer erfolgreiche Biogasbildung kann der Überdruck des Biovergasungsprozesses gegenüber der Atmosphäre konstant zwischen 0 bar und 10 bar liegen, vorzugsweise zwischen 0,01 und 7 bar.

Bei einer bevorzugten Weiterführung der Erfindung kann das gewonnene Biogas entfeuchtet und von CO₂ getrennt werden. Das derart gewonnene CO₂ kann gewinnbringend für verschiedene Zwecke herangezogen werden, beispielsweise als Schweißgas in der Metallindustrie oder dergleichen.

Erfindungsgemäß kann die CO₂- Trennung mittels Druckwechseladsorption erfolgen. Dadurch ist eine hohe Reinheit und ein sehr guter Heizwert des Biogases erzielbar.

Bei einer vorteilhaften Weiterbildung der Erfindung kann vorgesehen sein, dass das Biogas in der dritten Station einer Kraft-Wärme-Kopplung zugeführt wird. Die Kraft-Wärme-Kopplung ermöglicht sehr hohe Nutzungsgrade.

Bei einer weiteren Ausbildung der Erfindung kann vorsehen, dass das Biogas in ein Gasleitungsnetz eingespeist wird. Eine direkte Nutzung dieser regenerativen Energieform ist damit beispielsweise für Haushalte und Industriebetriebe möglich.

Die erfindungsgemäße Vorrichtung zur Erzeugung und Verteilung von Energie, bei der lignocellulosehaltige Rohstoffe verwertet werden, zeichnet sich aus durch eine erste Station, in welcher die Rohstoffe angeliefert werden, wobei in dieser ersten Station eine Vorbehandlung und Aufbereitung der Rohstoffe zu einem flüssigen Gärsubstrat erfolgt, eine zweite Station, in welcher das Gärsubstrat einem Biovergasungsprozess zugeführt wird, und eine dritte Station, in welcher das aus dem Biovergasungsprozess gewonnene Biogas in eine über größere Strecken transportierbare, bzw. leitbare Energieform gewandelt wird, insbesondere elektrische Energie, Wärmeenergie, oder chemische Energie.

Bei einer vorteilhaften weiterbildung der erfindungsgemäßen Vorrichtung kann vorgesehen sein, dass eine Sortier- und Reinigungsvorrichtung zur Reinigung der Rohstoffe und eine Aufschlussvorrichtung zum Aufschließen und Zerkleinern der Rohstoffe vorgesehen ist, welcher eine Aufbereitungseinrichtung nachgeschaltet ist für die Herstellung eines verflüssigten Gärsubstrates vermittels einer hydrolytischen Substratverflüssigung. Hierbei kann die Aufschlussvorrichtung eine Mahleinrichtung und/oder eine Vergleichmäßigungseinrichtung und/ oder eine Pumpeneinrichtung und/oder eine Ultraschalleinrichtung und/oder eine Auflösevorrichtung zur Herstellung eines Rohstoffbreis aufweisen. Die Mahleinrichtung kann einen Ein-oder Doppelscheibenrefiner und/oder einen Kegelrefiner und/oder eine Kugelmühle aufweisen. Die Auflösevorrichtung kann einen Behälter und eine Auflöseeinheit aufweisen. Hierbei kann der Behälter ein Sieb aufweisen zum Zwecke des Rückhaltens zu großer Rohstoffpartikel.

Bei einer vorteilhaften Weiterbildung der erfindungsgemäßen Vorrichtung kann vorgesehen sein, dass die Auflöseeinheit als Messer und/oder als Scheibenrotor und/oder als Schraubenrotor ausgeführt ist.

Bei einer vorteilhaften Weiterbildung der erfindungsgemäßen Vorrichtung kann vorgesehen sein, dass die Aufbereitungseinrichtung einen Behälter mit einer Rührvorrichtung aufweist. Hierbei kann die Aufbereitungseinrichtung eine Sensoreinrichtung zur Erfassung der elektrischen Leitfähigkeit und/oder des pH-Wertes und/oder eine Stoffdichtemesseinrichtung zur Erfassung der Dichte des Rohstoffbreis aufweisen. Von Vorteil kann die Aufbereitungseinrichtung mit einer Dosiereinrichtung für die Beigabe von Enzymen zum Rohstoffbrei versehen sein.

Von Vorteil kann bei der erfindungsgemäßen Vorrichtung weiterhin vorgesehen sein, dass eine Einrichtung zur Proteinrückgewinnung vorgesehen ist, welche die folgenden Merkmale aufweist: eine Pressvorrichtung zum Auspressen des geschnittenen und gemahlenen Rohstoffes; eine Filtervorrichtung zum Reinigen des derart gewonnenen Saftes; eine Vorrichtung zum Erhitzen des derart gewonnenen Saftes auf maximal 90°C, und eine Separatorvorrichtung zum Dekantieren der Proteine.

Weitere Zweckmäßigkeiten, Merkmale und Vorteile der Erfindung sind in den Unteransprüchen angegeben.

Im Folgenden wird ein Ausführungsbeispiel der Erfindung anhand der Zeichnung näher erläutert. Es zeigt:
- Fig. 1: ein Flussdiagramm, welches die Schritte des erfindungsgemäßen Verfahrens wiedergibt; und
- Fig.2: eine schematische Ansicht der erfindungsgemäßen Vorrichtung.

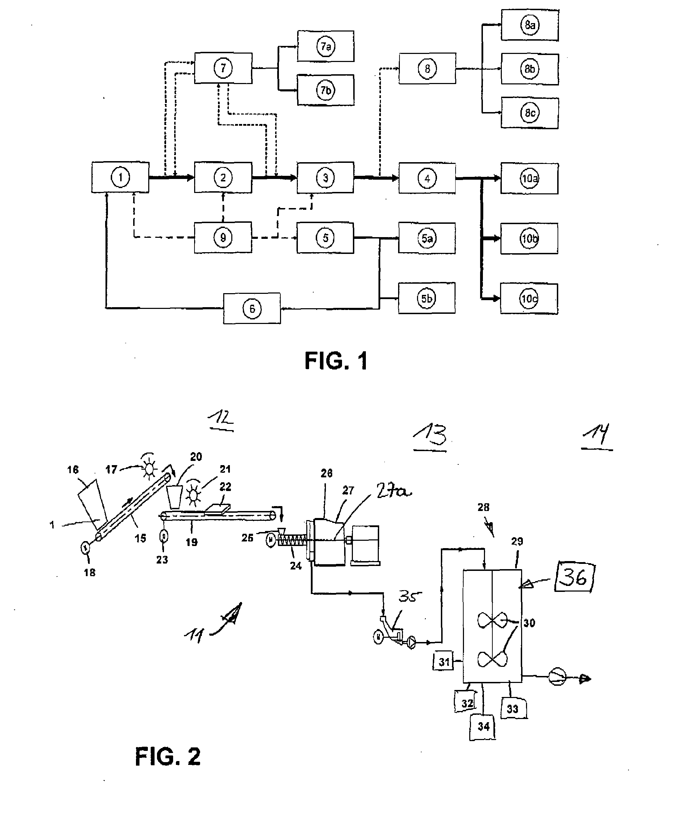

Gemäß Figur 1 erfolgen Vorbehandlung und Aufarbeitung 2 der Biomasse 1 in der Art und Weise, dass eine Oberflächenvergrößerung der Biomasse herbeigeführt wird, indem die Biomasse aufgeschlossen, d.h. vorbehandelt, sortiert und von Verunreinigungen befreit und zerkleinert wird. Angelieferte Biomasse 1, z.B. Gras, Silage, Holzschnitt wird auf eine Länge von 0,1 mm bis 10 mm, vorzugsweise auf 0,2 mm bis 3 mm gekürzt. Anschließend wird die Biomasse 1 zerfasert, bzw. fibrilliert, d.h. die Oberfläche der einzelnen Biomassepartikel 1 wird so verändert, dass die so erhaltene Oberfläche das zwei- bis 1000-fache der Ausgangsoberfläche beträgt. Dadurch entsteht eine erhöhte Angriffsfläche für die verschiedenen Bakterienstämme des Biogasprozesses 3 (Hydrolyse und Biogenese). Vorbehandlung und Aufbereitung 2 der Biomasse 1 erfolgen in festem und/oder flüssigem Zustand. Der Feststoffgehalt der Roh-Biomasse 1 liegt dabei für Gräser im Bereich von 15% bis 40%, für Körner im Bereich von 70% bis 95%, für Silagen im Bereich von 15% bis 40%, und für Wasserschnitt im Bereich von 2% bis 40%. Der Feststoffgehalt des eigentlichen Aufbereitungsprozesses 2 liegt im Bereich von 5% bis 50% Ts (Trockensubstanz) des verwendeten Biomasserohstoffes. Als Prozessaggregate zur Vorbehandlung 2 werden Mahlgeräte wie Ein- und/oder Doppelscheibenrefiner und/oder Kegelrefiner und/oder Kugelmühlen, die drucklos und/oder unter Druck und betreiben werden, eingesetzt. Ein weiteres Prozessaggregat, welches zur Vorbehandlung 2 eingesetzt werden kann, ist ein kontinuierliches und/oder diskontinuierliches Auflöseaggregat, bestehend aus einem Tank und aus einer Auflöseeinheit, die in Messerform und/oder Scheibenrotorform und/oder Schraubenrotorform ausgeführt ist, die gegebenenfalls mit einem Feinstoffabzug in Form eines Siebes ausgestattet ist, welches zu große Biomassepartikel 1 zurückhält. Auflöseeinheit und Mahlaggregat können auch in Kombination, d.h. ausgeführt als interne Rezirkulation in Reihenschaltung und/oder in Parallelschaltung eingesetzt werden. Bei einer weiteren Ausführung können ein und/ oder mehrere parallel und/oder in Reihe geschaltete Auflöseeinheiten und/oder Mahlaggregate verwendet werden. Die im Biomasserohstoff 1 eingelagerten Proteine können im Schritt 7 vor oder nach der Vorbehandlung 2 rückgewonnen werden, wobei der zerkleinerte bzw. zerfaserte Rohstoff ausgepresst wird. Der so gewonnene Saft wird gereinigt und auf maximal 90°C erhitzt, mit einem anschließenden Dekantieren der Proteine. Die gewonnenen Proteine können in der Pharmazie 7a oder in der Chemie 7b eingesetzt werden. Das Filtrat wird dem eigentlichen Biogasprozess 3 zugeführt. Eine Zugabe von Enzymen zu den Rohstoffen erfolgt vor oder während der Vorbehandlung 2 in einem Schritt 9, kann aber auch noch während des Biogasprozesses 3 stattfinden. Die Rohstofftemperatur liegt während des Vorbehandlungsprozesses im Eintrittsbereich zwischen -10°C und +50°C und zwischen +10°C und +90°C im Austrittsbereich, vorzugsweise zwischen +5°C und +50°C im Eintrittsbereich und zwischen +35°C und +85°C im Austrittsbereich, dabei kann von einer Temperaturerhöhung von bis zu 80°C, vorzugsweise um bis zu 50°C ausgegangen werden. Der Überdruck innerhalb der Vorrichtung zur Vorbehandlung im Schritt 2 gegenüber der Atmosphäre liegt zwischen 0 bar und 10 bar, vorzugsweise zwischen 0,025 und 5 bar. Der pH-Wert innerhalb der Auflöseeinheit liegt zwischen 4 und 12, vorzugsweise zwischen 4,5 und 7. Die anfallenden Prozesswässer 5 der Biogasanlage können in Düngemittel (flüssig/fest) im Schritt 5b, bzw. nach weiterer Behandlung in Trinkwasser gemäß Scritt 5a umgewandelt werden. Der Prozess der Vorbehandlung (Schritt 2) und/oder der Biogaserzeugung (Schritt 3) wird über den pH-Wert und/oder die elektrische Leitfähigkeit gesteuert. Die Biogaserzeugung gemäß Schritt 3 ist räumlich getrennt von der Vorbehandlung gemäß Schritt 2, der Transport des Rohstoffbreis erfolgt über Rohrleitungen oder mittels üblichen Transportmitteln wie beispielsweise Lastkraftwagen. Der pH-Wert des Gärsubstrates während der Biovergasung liegt zwischen 4 und 12, vorzugsweise zwischen 6 und 8. Der Überdruck innerhalb der zweiten Station während des Biovergasungsprozesses gegenüber der Atmosphäre liegt konstant zwischen 0 bar und 10 bar, vorzugsweise zwischen 0,01 und 7 bar. Die eingesetzte Energie liegt zwischen 5 kWh/tTs (Trockensubstanz) bis 200 kWh/tTs, vorzugsweise zwischen 10 kWh/tTs und 125 kWh/tTs. Der Biogaserzeugungsprozess gemäß Schritt 3 kann in einem oder in mehreren Reaktoren stattfinden, wobei diese Reaktoren parallel und/oder in Reihe und oder als parallel- und/oder Reihenkombination geschaltet sind. Die Einstellung des pH-Wertes der Vorbehandlungs- und/oder des Biogasprozesses gemäß Schritt 3 kann mit Natrium- und/oder Kalziumhydroxid mit niedriger und/oder mittlerer und/oder hoher Reaktivität betreiben werden. Das produzierte Biogas gemäß Schritt 4 enthält neben Methan noch CO₂ in einem Anteil von 30 bis 70%. Das Biogas 4 wird durch Druckwechseladsorption 8 von CO₂ getrennt, und es erfolgt eine Entfeuchtung. Das CO₂ wird in weiterer Folge entsorgt (gemäß Schritt 8a) oder industriellen Anwendungen gemäß Schritt 8b (Schweißgas, Gas zur Erzeugung von Trockenes, Kühlmittel) und/oder agrartechnischen Anwendungen gemäß Schritt 8c (z.B. Begasen von Gewächshäusern mit CO₂ für schnellere Reifung und rascheres Wachstum von Nahrungs- und Gebrauchspflanzen) zugeführt. Das verbleibende Biogas 4 ist durch diese Reinigung energetisch von höherem Wert, und kann nun optimal auf verschiedene Arten zur Energieproduktion (gemäß Schritte 10a, 10b, 10c) genutzt werden: Ein Teil des Gases kann in das Gasleitungsnetz gemäß Schritt 4c eingespeist werden, während ein Teil des Gases zur Befeuerung eines Heizkraftwerkes herangezogen wird. In diesem Heizkraftwerk erfolgt eine Kraft-Wärme-Kopplung, d.h. es wird aus dem Biogas 4 elektrische Energie gemäß Schritt 10a erzeugt, und die überschüssige Wärmeenergie gemäß Schritt 10b der Anlage wird in das Fernwärmenetz gemäß Schritt 10c eingeleitet. Der Gärrest gemäß Schritt 6 kann als Dünger in der Landwirtschaft verwendet werden, wodurch der biologische Kreislauf geschlossen wird.

Figur 2 zeigt ein Ausführungsbeispiel einer erfindungsgemäßen Vorrichtung 11 zur Erzeugung und Verteilung von Energie, bei der lignocellulosehaltige Rohstoffe 1 verwertet werden. Diese Biogasanlage 1 hat bei einem Stoffdurchsatz von etwa 30 t pro Tag, einem speziellen Energieeintrag von geringer als 15 kWh pro Tag, und einer installierten Leistung von etwa 75 kW eine Ertragsleistung von etwa 500 kW. Es ist eine erste Station 12 vorgesehen, in welcher die Rohstoffe 1 angeliefert werden, wobei in dieser ersten Station 12 bereits eine Vorbehandlung und Aufbereitung der Rohstoffe 1 zu einem flüssigen Gärsubstrat erfolgt. Die Vorrichtung 11 umfasst ferner eine zweite Station 13, in welcher das Gärsubstrat einem Biovergasungsprozess zugeführt wird, und eine dritte Station 14, in welcher das aus dem Biovergasungsprozess gewonnene Biogas in eine über größere Strecken transportierbare, bzw. leitbare Energieform gewandelt wird, insbesondere elektrische Energie, Wärmeenergie, oder chemische Energie.

Die erste Station 12 hat eine Sortier- und Reinigungsvorrichtung zur Reinigung und Egalisierung der Rohstoffe 1 und eine Aufschlussvorrichtung zum Aufschließen und Zerkleinern der Rohstoffe. Die erste Staion 12 weist ein Schräg-Transport-Stollen Förderband 15 mit einer speziellen Einlaßschnurre 16, einem Egalisiersonnenrad 17 inkl. Aufsteckgetriebemotor 18 und Frequenzumrichter, ein Horizontal-Transport-Stollen Förderband 19 mit einer speziellen Einlaßschnurre 20, einem Egalisiersonnenrad 21 mit einer Bandwiegeeinrichtung 22, und einem Aufsteckgetriebemotor 23 und Frequenzumrichter auf. Über eine Einspeiseschnecke 24 mit einer weiteren speziellen Einlaßschnurre 25 inkl. Riemenantrieb, Antriebmotor und Frequenzumrichter wird das Rohstoffgut der Aufschlussvorrichtung 26 zugeführt. Die Aufschlussvorrichtung 26 weist eine Mahleinrichtung 27 auf, in dessen Mahlraum ein aus hochfestem, rostbeständigem Turbinenwerkstoff gefertigter Rotor 27a mit einer händisch erfolgenden Rotorverstellung gelagert ist, wobei die im Maschinenkörper angeordnete Lagereinheit im Bereich der beiden Lagerstellen in speziellen Gleitführungen am kompletten Umfang im Maschinenrahmen geführt ist. Die Mahleinrichtung 27 kann einen Ein-oder Doppelscheibenrefiner und/oder einen Kegelrefiner und/oder eine Kugelmühle aufweisen. Es ist eine Aufbereitungseinrichtung 28 für die Herstellung eines verflüssigten Gärsubstrates vermittels einer hydrolytischen Substratverflüssigung nachgeschaltet. Die Aufbereitungseinrichtung 28 weist einen Behälter 29 mit einer Rührvorrichtung 30 auf. Die Aufbereitungseinrichtung 28 besitzt eine Sensoreinrichtung 31 zur Erfassung der elektrischen Leitfähigkeit und/oder des pH-Wertes und eine Stoffdichtemesseinrichtung 32 zur Erfassung der Dichte des Rohstoffbreis. Die Aufbereitungseinrichtung 28 besitzt eine Dosiereinrichtung 33 für die Beigabe von Enzymen zum Rohstoffbrei. Es ist eine Einrichtung 34 zur Proteinrückgewinnung vorgesehen, welche eine Pressvorrichtung zum Auspressen des geschnittenen und gemahlenen Rohstoffes, eine Filtervorrichtung zum Reinigen des derart gewonnenen Saftes, eine Vorrichtung zum Erhitzen des derart gewonnenen Saftes auf maximal 90°C, und eine Separatorvorrichtung zum Dekantieren der Proteine aufweist. Eine Pumpeneinrichtung 35 mit einer MC-Pumpe (Mittelkonsistenz-Pumpe) als Spezialausführung einer Zentrifugalpumpe dient dem Transport des Biogutes an die Auflösevorrichtung 28 zur Herstellung des Rohstoffbreis. Zur Erhöhung des organischen Trockensubstanzabbaus ist eine Ultraschalleinrichtung 36 vorgesehen. Ziel der Anwendung mit Ultraschall ist die akustische Erzeugung von Kavitation in der Substrat-Suspension. Es bilden sich mikroskopisch kleine Hohlräume in der Flüssigkeit (Kavitation). Die bei der Ultraschall-Kavitation ausgelösten extremen Scherkräfte führen zu einem Aufschluß der biologischen Zellen. Die Zellwände werden zerstört und das zellinnere geht in Lösung. Auf diese Weise kann ein Abbau der organischen Trockensubstanz bis zu 90 bis 95 % und damit in der Folge ein quantitativer Anstieg an methanhaltigem Biogas von 15 bis 30 % abhängig von der Qualität des Substrats erreicht werden.

## Patentansprüche

1. Verfahren zur Erzeugung und Verteilung von Energie, bei dem lignocellulosehaltige Rohstoffe (1) verwertet werden,
**dadurch gekennzeichnet, dass**
in einer ersten Station die Rohstoffe (1) angeliefert werden, und in dieser ersten Station eine Vorbehandlung und Aufbereitung (2) dieser Rohstoffe (1) zu einem flüssigen Gärsubstrat erfolgt,
in einer zweiten Station das Gärsubstrat einem Biovergasungsprozess (3) zugeführt wird, und
in einer dritten Station das aus dem Biovergasungsprozess (3) gewonnene Biogas (4) in eine über größere Strecken transportierbare, bzw. leitbare Energieform gewandelt wird, insbesondere elektrische Energie (4a), Wärmeenergie (4b), oder chemische Energie (4c).

2. Verfahren nach Anspruch 1, **dadurch gekennzeichnet, dass** die zweite Station von der ersten räumlich getrennt ist.

3. Verfahren nach Anspruch 1 oder 2, **dadurch gekennzeichnet, dass** die Rohstofftemperatur während der Vorbehandlung (2) im Eintrittsbereich zwischen -10°C und +50°C liegt und zwischen +10°C und +90°C im Austrittsbereich, vorzugsweise zwischen +5°C und +50°C im Eintrittsbereich und zwischen +35°C und +85°C im Austrittsbereich.

4. Verfahren nach einem der vorhergehenden Ansprüche, **dadurch gekennzeichnet, dass** die Aufbereitung (2) der vorbehandelten Rohstoffe (1) durch hydrolytische Verflüssigung erfolgt, wobei die hydrolytische Verflüssigung insbesondere durch Enzyme (9) und/oder thermophile Bakterienkulturen herbeigeführt wird.

5. Verfahren nach einem der vorhergehenden Ansprüche, **dadurch gekennzeichnet, dass** der pH-Wert des Gärsubstrates während des Biovergasungsprozesses (3) zwischen 4 und 12 liegt, vorzugsweise zwischen 6 und 8.

6. Vorrichtung zur Erzeugung und Verteilung von Energie, bei der lignocellulosehaltige Rohstoffe (1) verwertet werden, **gekennzeichnet durch**:
a) eine erste Station, in welcher die Rohstoffe (1) angeliefert werden, wobei in dieser ersten Station eine Vorbehandlung und Aufbereitung (2) der Rohstoffe (1) zu einem flüssigen Gärsubstrat erfolgt,
b) eine zweite Station, in welcher das Gärsubstrat einem Biovergasungsprozess (3) zugeführt wird, und
c) eine dritte Station, in welcher das aus dem Biovergasungsprozess (3) gewonnene Biogas (4) in eine über größere Strecken transportierbare, bzw. leitbare Energieform gewandelt wird, insbesondere elektrische Energie (4a), Wärmeenergie (4b), oder chemische Energie (4c).

7. Vorrichtung nach Anspruch 6, **dadurch gekennzeichnet, dass** eine Sortier- und Reinigungsvorrichtung zur Reinigung der Rohstoffe(1) und eine Aufschlussvorrichtung zum Aufschließen und Zerkleinern der Rohstoffe (1) vorgesehen ist, welcher eine Aufbereitungseinrichtung nachgeschaltet ist für die Herstellung eines verflüssigten Gärsubstrates vermittels einer hydrolytischen Substratverflüssigung.

8. Vorrichtung nach Anspruch 7, **dadurch gekennzeichnet, dass** die Aufschlussvorrichtung eine Mahleinrichtung und/oder eine Vergleichmäßigungseinrichtung und/oder eine Pumpeneinrichtung und/oder eine Ultraschalleinrichtung und/oder eine Auflösevorrichtung zur Herstellung eines Rohstoffbreis aufweist.

9. Vorrichtung nach Anspruch 8, **dadurch gekennzeichnet, dass** die Mahleinrichtung einen Ein-oder Doppelscheibenrefiner und/oder einen Kegelrefiner und/oder eine Kugelmühle aufweist.

10. Vorrichtung nach einem der Ansprüche 8 oder 9, **dadurch gekennzeichnet, dass** die Auflösevorrichtung einen Behälter und eine Auflöseeinheit aufweist.

11. Vorrichtung nach einem der Ansprüche 8 bis 10, **dadurch gekennzeichnet, dass** der Behälter ein Sieb aufweist zum Zwecke des Rückhaltens zu großer Rohstoffpartikel.

12. Vorrichtung nach einem der Ansprüche 8 bis 11, **dadurch gekennzeichnet, dass** die Auflöseeinheit als Messer und/oder als Scheibenrotor und/oder als Schraubenrotor ausgeführt ist.

13. Vorrichtung nach einem der Ansprüche 6 bis 12, **dadurch gekennzeichnet, dass** die Aufbereitungseinrichtung einen Behälter mit einer Rührvorrichtung aufweist.

14. Vorrichtung nach-einem der Ansprüche 6 bis 13, **dadurch gekennzeichnet, dass** die Aufbereitungseinrichtung eine Sensoreinrichtung zur Erfassung der elektrischen Leitfähigkeit und/oder des pH-Wertes und/oder eine Stoffdichtemesseinrichtung zur Erfassung der Dichte des Rohstoffbreis aufweist.

15. Vorrichtung nach einem der Ansprüche 6 bis 14, **dadurch gekennzeichnet, dass** die Aufbereitungseinrichtung mit einer Dosiereinrichtung für die Beigabe von Enzymen (9) zum Rohstoffbrei versehen ist.
